Europäisches Patentamt

European Patent Office

Office européen des brevets

(19)

(11) EP 0 506 664 B1

## (12) EUROPEAN PATENT SPECIFICATION

(45) Date of publication and mention
of the grant of the patent:
**25.11.1998 Bulletin 1998/48**

(21) Application number: **90914255.6**

(22) Date of filing: **17.09.1990**

(51) Int Cl.6: **G06F 17/50**
// G06F159/00, G01N33:00

(86) International application number:
**PCT/SE90/00593**

(87) International publication number:
**WO 91/04543 (04.04.1991 Gazette 1991/08)**

(54) **COMPUTER GRAPHICS FOR MOLECULAR DESIGN**

COMPUTERGRAPHIK FÜR MOLEKÜLENTWURF

INFOGRAPHIE APPLIQUEE A LA CONCEPTION MOLECULAIRE

(84) Designated Contracting States:
**DE GB**

(30) Priority: **18.09.1989 SE 8903055**

(43) Date of publication of application:
**07.10.1992 Bulletin 1992/41**

(73) Proprietors:
• **BRINCK, Nils Tore**
**S-791 91 Falun (SE)**
• **SJÖBERG, Per**
**S-691 41 Karlskoga (SE)**

(72) Inventors:
• **BRINCK, Nils Tore**
**S-791 91 Falun (SE)**
• **SJÖBERG, Per**
**S-691 41 Karlskoga (SE)**

(74) Representative: **Brinck, Curt Edvard**
**Ingenjörsfirma Curt Brinck**
**Hedens gard 50**
**791 91 Falun (SE)**

(56) References cited:
**WO-A-88/01775**

• **J. Mol. Graphics, Vol. 8, June 1990 P SJOBERG:
"The use of the electrostatic potential at the
molecular surface in recognition interactions:
Dibenzo-p-dioxins and related systems", see
page 81 - page 90.**
• **J. Phys. Chem., Vol. 94, 1990 P SJOBERG: "Use
of the Electrostatic Potential at the Molecular
Surface To Interpret and Predict Nucleophilic
Processes", see page 3959 - page 3961.**
• **Croatica Chemica acta, Vol. 57, No. 5, 1984 D
HADZI et al.: "The Molecular Electrostatic
Potential as a Determinant of Receptor-Drug
Recognition", see page 1065 - page 1074. See
page 1070.**
• **Pharmaceutical Research, Vol. 3, No. 4, 1986 T.
P. LYBRAND et al.: "Conformation and
Electrostatic Potential Surfaces of Opiates:
Relationship to mu- and kappa-site Binding",
see page 218 - page 224. See page 220, second
paragraph.**
• **Journal of Computational Chemistry, Vol. 9, No.
5, 1988 G. A. ARTECA et al.: "Shape
Characterization of Some Molecular Model
Surfaces", see page 554 - page 563.**
• **Drug Information Journal, Vol. 17, 1983 D. B.
BOYD: "Quantum mechanics in drug design:
Methods and applications", see page 121 - page
131.**

## Description

This invention will describe a method to graphically describe the surface of a molecule and on this surface present calculated molecular properties to be able to study, develop and manufacture molecules, preferably biologically active molecules for the pharmaceutical industry.

The surface of a molecule is often described with help of the Van der Waals model. However this model does not show the change of the electronic density around atoms when atoms bind to molecules. It is instead better to let the surface be defined as an envelope having constant electronic density. This surface does better describe the very electronic density that makes a molecule unique.

It is known that a biologically active molecule often binds to specific receptors in the cells. This binding is often an effect of the receptors and the molecules having complementary forms and complementary qualities, for example electrostatic potential. Positive surfaces on the receptor bind to negative surfaces on the active molecule and vice versa. In the same way the average local ionization energy described below works, i.e. surfaces with a high ionization energy bind to surfaces with a low energy according to the theory of electrophilic-nucleophilic interaction. Using the average ionization potential on the surfaces of the molecule open a new possibility to study the electrophilic-nucleophilic interaction.

For industrially designing a new drug, one has to first find the part of the cell that is the receptor. After that one has to make a list of molecules able to bind to the receptor and thus having the desired biological effect. To determine the activity of the molecules, one must first synthesize them. This is often a very arduous task. After that, one has to make tests on animals to determine the biological activity. This is also very expensive and usually takes a long time. To day there are instruments to determine which molecules have the best qualifications to bind to the receptor. The invention allows to reduce the number of synthetizations and the development work only to a few molecules. That would lead to big economic effects for many drugs producing companies.

Attempts have been made to avoid this development labour and costs. For instance, a paper by D. Hadzi et al., (CROATICA CHEMICA ACTA, vol. 57, no. 5, pages 1065-1074, 1984, "The Molecular Electrostatic potential as a Determinant of Receptor-Drug Recognition") describes methods for calculating the electrostatic potential V at a point P, which involves determining "the molecular wave function", using inter alia "a standard ab-initio method".
The electrostatic values in the region surrounding a molecule are calculated at grid points located on a three-dimensional surface whereby equipotential 3-D surfaces are determined.

It has now been shown that it is possible to get a better applicable graphical picture of molecules and then, for example, be able to adjust them to receptors in a cell by graphically showing the molecule as an envelope having given electronic density and to this envelope associating specified molecular properties.

The invention will be described in more detail below, with examples, and with help of the enclosed drawings, where

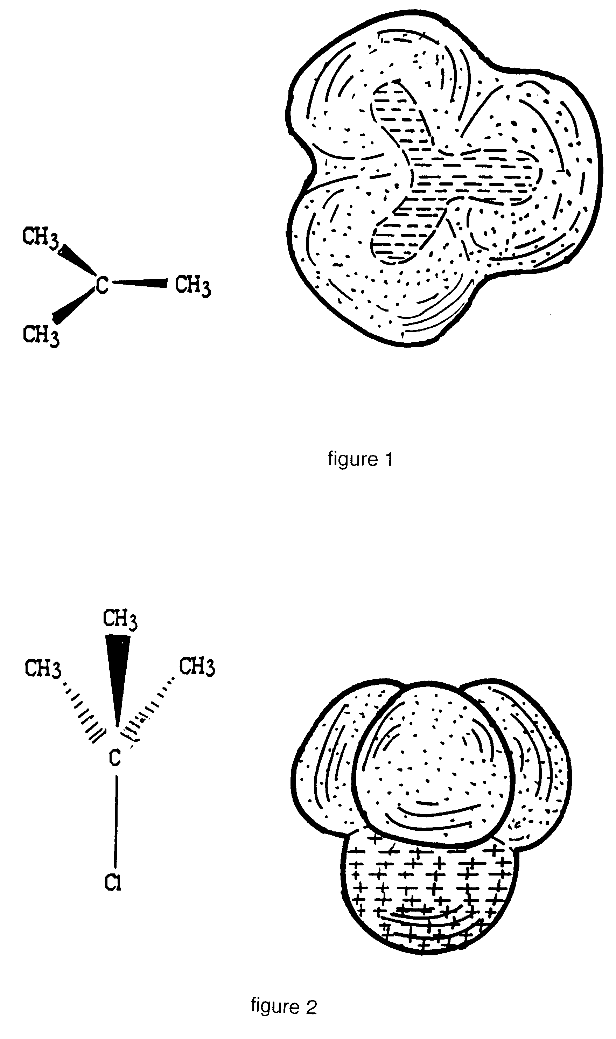

figure 1 shows the tert butyl chloride molecule
-"- 2 -"- -"- -"- -"- -"- -"- from another view
-"- 3 -"- -"- methyl chloride molecule
-"- 4 -"- -"- tetra chloro dibenzodioxine molecule
-"- 5 -"- -"- dibenzodioxine molecule and
-"- 6 -"- -"- aniline molecule

By using an ab-initio or a semi empirical quantum chemical calculation process it is possible to get an approximate wave function for any molecule. The surface of the molecule is defined as an envelope having constant or near constant electron density. The surface will be generated by calculating so called "grid points", having equal electron density. For making that, one will use a repeated algorithm, where the electron density and its gradient in every point are calculated analytically from the wave function. The normal to this surface has in every point the same direction as the gradient of the electron density in this point. The normal is thus obtained by normalization of the gradient. At every point of the surface there are a number of molecular properties, calculated from the wave function, for example:

the electrostatic potential
the electrostatic field perpendicular to the surface
the electrostatic potential polarized by any point charge
the HOMO density and
the average local ionization energy

All properties with exception of the average local ionization energy are well known and described in the literature. The average local ionization energy "I" at the coordinate "r" is defined below:

$$I = -\frac{\sum p_i(r).e_i}{p(r)}$$

where

"$p_i(r)$" is the density contribution from orbital "i" at "r",
"$p(r)$" is the density of the surface (usually $\emptyset. \, \emptyset\emptyset\emptyset$ electrons/bohr$^3$)
"$e_i$" is the orbital energy for orbital "i".

The summation is made over all occupied orbitals.
With help of computer graphics the three dimensional surface is displayed color coded according to the value of one of the molecular properties above.

Example 1.

This example demonstrates, how the invention can be used to predict nucleophilic attacks. The approach is that surfaces with high electrostatic potential are highly susceptible for nucleophilic attacks. Three pictures have been produced in a computer according to the invention. The pictures on the screen have been transferred to three figures, figure 1-3. The colors are of course lost in the black and white print shown here and some of the three dimensional impressions produced by a computer color screen are lost.

Figure 1 shows tert butyl chloride similar to the picture.
Figure 2 shows also tert butyl chloride but from another direction
Figure 3 shows in the same way methyl chloride.

The areas marked with half-broken lines have a potential higher than +21 kcal/mole, dotted areas have a potential between +21 and Ø kcal/mole and +dotted areas have a potential below Ø kcal/mole.
But tert-butyl chloride is much less reactive towards nucleophilic attack due to the steric hinderence at the substituted carbon.

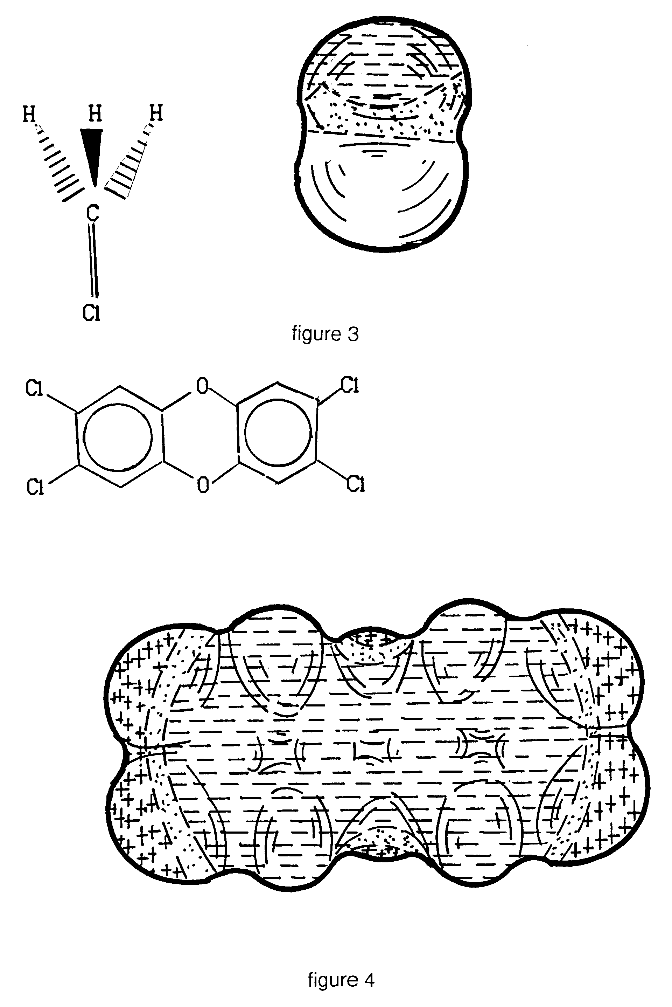

Figure 3 shows methyl chloride. The coding is the same as for tert butyl chloride. Carbon shows a big surface with high electrostatic potential and is consequently highly sensitive for a nuclear attack.

Example 2.

Figure 4 shows the molecular surface of a tetra chloro-dibenzodioxine molecule. The area with half-broken lines represents an electrostatic potential on the surface less than Ø kcal/mol, dotted areas represents a potential between Ø and -1Ø kcal/mol and +dotted areas below -1Ø kcal/mole. This molecule is known to be highly toxic.

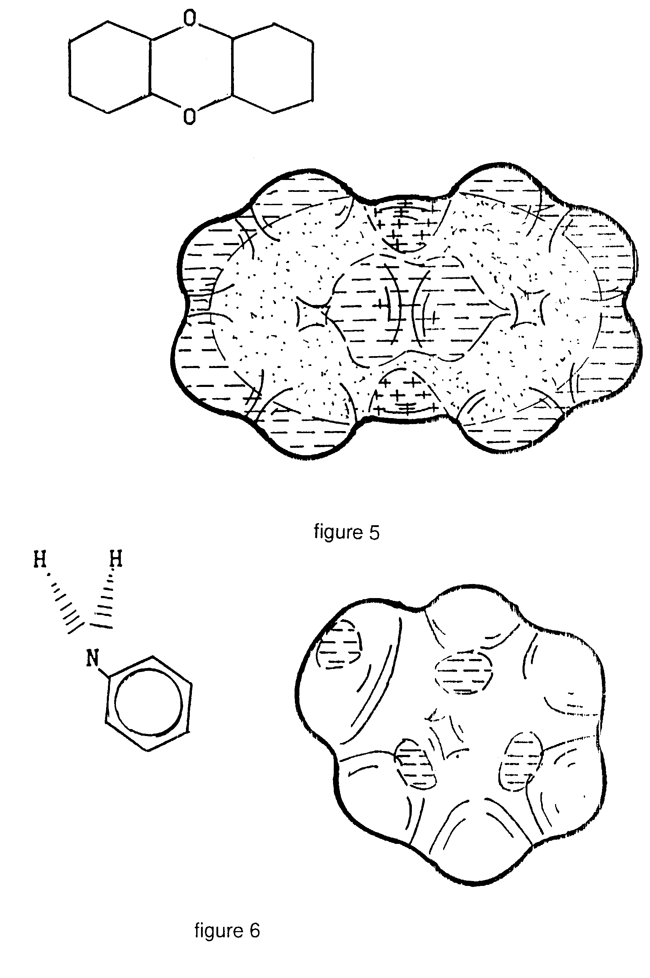

Figure 5 shows the molecular surface of dibenzodioxine, coded in the same way as figure 3. This molecule is rather similar to tetra chloro dibenzodixoine but is not toxic. It is noted from the picture that the molecule shows a negative potential on most of its surface in contrast to tetra chloro dibenzodioxin. Scientific publications teach that biological activity of dioxins depends on the electrostatic potential. This example will show that this invention displays better than other corresponding methods the difference between the potentials and the forms of molecules.

Example 3.

Figure 6 shows the molecular surface of aniline and the average ionization energy on the surface. The regions marked with half-broken lines have the lowest energy. The tendency of the molecule to transport electrons to an attacking electrophilic molecule is here maximized. That corresponds with the experimentally shown picture of aniline as orto-para directing by electrophilic substitution. It is also a surface marked with half-broken lines over the free electronic pair of nitrogen. By this function the molecule will become protonized. It is also a type of nuclear attack. There are no other methods that so clearly point out the electrophilic attack sites on a molecule.

**Claims**

1. A quantum chemical method for molecular design comprising the steps of gradually selecting a number of molecules having probably desirable properties, use an ab-inito or similar quantum chemical method for getting an approximate wave function for the molecule in question, said method being characterised by the further steps of using a repeating algorithm or similar methods for analytically calculating equidistant grid points from the wave function and then defining the surface of the molecule as an envelope of constant or near constant electronic density and with help of three dimensional computer graphics illustrating quantum chemically calculated molecular properties on this surface and then comparing this picture with pictures of corresponding calculated molecules having known desired biological activity or with molecules of the receptor in the cell.

2. The quantum chemical method according to claim 1 **characterized in** that the illustrated molecular property is the electrostatic potential.

3. The quantum chemical method according to claim 1 **characterized in** that the illustrated molecular property is the average local ionization energy.

4. The quantum chemical method according to claim 1 **characterized in** that the illustrated molecular property is the electrostatic field perpendicular to the surface.

5. The quantum chemical method according to claim 1 **characterized in** that the molecular property is the HOMO density.

6. The quantum chemical method according to claim 1 **characterized in** that the molecule in question is biologically active.

**Patentansprüche**

1. Arten, wie quantenchemische Methoden für Molekül-Design anzuwenden sind, zeichnen sich dadurch aus, dass man successiv eine Anzahl Moleküle mit möglichen wünschenswerten Eigenschaften auswählt und auf diese ein ab-inito oder eine andere quantenchemische Methode anwendet um eine approximative Kurvenfunktion für das Molekül zu erhalten; dass man einen repetierenden Algorithmus oder ähnliches anwendet um aus dieser Kurvenfunktion eqvidistante Punkte analytisch zu berechnen und die Oberfläche des Moleküls zu definieren als eine Hülle aus konstanter oder beinahe konstanter Elektrodensität; dass man auf dieser Oberfläche mit dreidimensioneller Grafik die quantenchemisch berechnet gesuchten Eigenschaften veranschaulicht und schliesslich diese Bilder mit entsprechenden Berechnungen von Molekülen mit bekannt biologischer Aktivität oder mit entsprechender Berechnung von dem Rezeptor des Moleküls in der Zelle vergleicht.

2. Diese Arten, wie nach Bedingung 1, zeichnen sich dadurch aus, dass die molekulare Eigenschaft elektrostatisches Potential ist.

3. Diese Arten, wie nach Bedingung 1, zeichnen sich dadurch aus, dass die molekulare Eigenschaft die durchschnittliche Ionisationsenergi ist.

4. Diese Arten, wie nach Bedingung 1, zeichnen sich dadurch aus, dass die molekulare Eigenschaft das elektrostatische Feld winkelrecht zur Oberfläche ist.

5. Diese Arten, wie nach Bedingung 1, zeichnen sich dadurch aus, dass die molekulare Eigenschaft die HOMO-Densität ist.

6. Diese Arten, wie nach Bedingung 1-4, zeichnen sich dadurch aus, dass der Stoff biologisch aktiv ist.

**Revendications**

1. Méthode pour utiliser les méthodes chimiques quantiques pour la conception de molécules, caractérisée en ce que l'on sélectionne successivement un certain nombre de molécules ayant de probables caractéristiques souhaitables et que l'on applique sur ces molécules une méthode ab-inito ou autre méthode chimique quantique pour obtenir une fonction d'onde approximative pour la molécule, et que l'on utilise un algorithme répétitif ou similaire pour calculer à partir de la fonction d'onde de façon analytique des points grille équidistants, pour définir la surface de la molécule comme une enveloppe de densité électronique constante ou quasi constante pour représenter, sur cette surface, à l'aide de graphique tridimensionnelle les caractéristiques recherchées calculées de façon chimique quantique et ensuite comparer ces images avec des calculs correspondants sur molécules avec activité biologique désirée connue ou avec un calcul correspondant sur le récepteur du molécule dans la cellule.

2. Méthode selon la revendication 1 caractérisée en ce que la caractéristique moléculaire est le potentiel électrostatique.

3. Méthode selon la revendication 1 caractérisée en ce que la caractéristique moléculaire est l'énergie moyenne d'ionisation.

4. Méthode selon la revendication 1 caractérisée en ce que la caractéristique moléculaire est le champ électrostatique perpendiculaire à la surface.

5. Méthode selon la revendication 1 caractérisée en ce que la caractéristique moléculaire est la HOMO-densité.

6. Méthode selon les revendications 1-4 caractérisée en ce que la matière est biologiquement active.

CH₃ ─ C ═ CH₃
       |
      CH₃

figure 1

figure 2

figure 3

figure 4

figure 5

figure 6